# EUROPÄISCHE PATENTSCHRIFT

(11) **EP 1 175 578 B1**
(45) Veröffentlichungstag und Bekanntmachung des Hinweises auf die Patenterteilung: **17.12.2003**
(21) Anmeldenummer: 01907228.9
(22) Anmeldetag: 22.02.2001
(51) Int. Cl.: F16L 25/00

(54) **VERBINDUNGSANORDNUNG FÜR LEITUNGEN**
CONNECTION ASSEMBLY FOR CONDUITS
DISPOSITIF DE RACCORDEMENT POUR CONDUITES

(30) Priorität: 23.02.2000 AT 2792000
(43) Veröffentlichungstag der Anmeldung: 30.01.2002
(73) Patentinhaber: Korpan, Nikolai, 1190 Wien (AT); Zharkov, Jaroslav, Kiew, 252146 (UA); Hochwarter, Gerhard, 1210 Wien (AT)
(72) Erfinder: Korpan, Nikolai, 1190 Wien (AT); Zharkov, Jaroslav, Kiew, 252146 (UA); Hochwarter, Gerhard, 1210 Wien (AT)
(74) Vertreter: Babeluk, Michael, Dipl.-Ing. Mag.
(86) Internationale Anmeldenummer: AT0100045
(87) Internationale Veröffentlichungsnummer: WO01063165

(56) Entgegenhaltungen:
- WO-A-98/15772
- CH-A- 273 781
- DE-C- 953 676
- FR-A- 2 341 094
- GB-A- 2 140 115
- US-A- 4 924 679

## Beschreibung

Die Erfindung betrifft eine Verbindungsanordnung für Leitungen, die koaxial ineinander angeordnet sind, um den Hinstrom und Rückstrom eines kryogenen Mediums aufzunehmen, mit einer ersten und einer zweiten Kupplungshälfte, die dichtend miteinander verbunden werden können, wobei eine innere Leitung in jeder Kupplungshälfte in einer ersten konusförmigen Dichtfläche endet, und wobei eine die innere Leitung umgebende äußere Leitung in jeder Kupplungshälfte in einer weiteren konusförmigen Dichtfläche endet, die radial außerhalb der ersten konusförmigen Dichtfläche angeordnet ist. Verbindungsanordnungen zwischen den koaxialen Leitungen für den Hinstrom und den Rückstrom eines kryogenen Mediums werden im medizinischen Bereich in der Kryochirurgie benötigt. Mit einer solchen Leitung wird beispielsweise ein Kryoapplikator mit einem Kryoinstrument verbunden, wobei wesentlich ist, dass die Verbindung lösbar ist. Kryochirurgische Geräte werden in der Krebsbehandlung aber auch in der Allgemeinchirurgie, in der Gynäkologie, im HNO-Bereich, in der Kieferchirurgie, in der Orthopädie, in der experimentellen Medizin, in der Veterinärmedizin, in der Phytopathologie und dergleichen eingesetzt.

Bei bekannten Erfindungen dieser Art erfolgt das Abdichten eines kryogenen Mediums mit einer Zwischenlage, zum Beispiel aus Kupfer. Nachteilig ist jedoch, dass eine solche Zwischenlage nur einige wenige Male verwendet werden kann und danach ersetzt werden muss.

Aus der FR 2 466 697 A ist eine Verbindungsanordnung bekannt, die diese Nachteile im Wesentlichen vermeidet. Es ist jedoch häufig erforderlich, auch eine elektrische Verbindung zwischen den Kryogeräten zu schaffen, die separat hergestellt wird. Diese zusätzliche Verbindung ist aufwändig und erschwert die Handhabung der einzelnen Teile. Auch bei weiteren Lösungen, wie sie in der WO 99/47845 A oder der US 4,772,050 A beschrieben sind, muss eine eventuelle elektrische Verbindung getrennt hergestellt werden.

Aufgabe der vorliegenden Erfindung ist es diese Nachteile zu vermeiden und zusätzlich zu den Verbindungen der Leitungen für das kryogene Medium eine einfache Möglichkeit einer elektrischen Verbindung zu schaffen.

Erfindungsgemäß werden diese Aufgaben dadurch gelöst, dass innerhalb der weiteren konusförmigen Dichtfläche in jeder Kupplungshälfte mindestens ein elektrischer Kontakt vorgesehen ist. Durch die erfindungsgemäße Anordnung ist der elektrische Kontakt geschützt und sicher gegenüber der Umgebung isoliert. Die Handhabung ist besonders einfach, da der elektrische Kontakt bei der Verbindung der Kupplungshälften automatisch hergestellt wird, ohne dass besondere Maßnahmen zu beachten sind.

Konstruktiv besonders günstig ist es, wenn die elektrischen Kontakte koaxial zu den Dichtflächen angeordnet sind. Auf diese Weise ist es nicht erforderlich, die beiden Kupplungshälften bei der Verbindung in Rotationsrichtung in Übereinstimmung zu bringen.

Eine besonders sichere elektrische Verbindung kann dadurch erreicht werden, dass die elektrischen Kontakte im Wesentlichen zylindrisch ausgebildet sind. Auf diese Weise wird bei den vorliegenden niederen Temperaturen eine sichere elektrische Verbindung erreicht.

Eine besonders günstige hydraulische Verbindung kann dadurch geschaffen werden, dass die erste Dichtfläche auf einem Dichtelement angeordnet ist, das in Axialrichtung verschiebbar und federnd vorgespannt mit der inneren Leitung verbunden ist. Die Handhabung der Verbindungsanordnung wird dabei insbesondere erleichtert, wenn die Kupplungshälften durch einen Bajonettverschluss verbindbar sind, wobei es besonders günstig ist, wenn der Bajonettverschluss eine Hülse aufweist, die durch eine Feder gegenüber einer Kupplungshälfte vorgespannt ist.

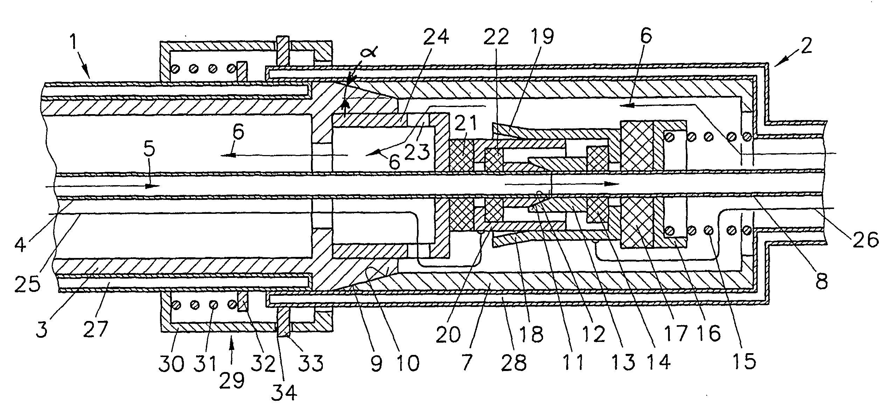

In der Folge wird die Erfindung anhand der in der Figur dargestellten Ausführungsvariante näher erläutert. Die Figur zeigt eine erfindungsgemäße Verbindungsanordnung im Axialschnitt.

Die erfindungsgemäße Verbindungsanordnung besteht aus einer ersten Kupplungshälfte 1 und einer zweiten Kupplungshälfte 2, die im verbundenen Zustand dargestellt sind. Die erste Kupplungshälfte 1 besitzt eine äußere Leitung 3, innerhalb der konzentrisch eine innere Leitung 4 angeordnet ist. Die innere Leitung 4 dient dem Hinstrom des kryogenen Mediums in der Richtung des ersten Pfeils 5, während die äußere Leitung 3 den Rückstrom in Richtung des zweiten Pfeils 6 aufnimmt. Die zweite Kupplungshälfte 2 besitzt in analoger Weise eine äußere Leitung 7 und eine innere Leitung 8 für den Hinstrom beziehungsweise Rückstrom des kryogenen Mediums. Wenn die erste und die zweite Kupplungshälfte 1, 2 verbunden sind, liegen eine konusförmige Dichtfläche 9 am Ende der äußeren Leitung 3 der ersten Kupplungshälfte 1 und eine konusförmige Dichtfläche 10 an dem Ende der äußeren Leitung 7 der zweiten Kupplungshälfte 2 dichtend aneinander an. Am Ende der inneren Leitung 4 der ersten Kupplungshälfte 1 ist weiters eine konusförmige Dichtfläche 11 vorgesehen, die mit einer weiteren konusförmigen Dichtfläche 12 zusammenwirkt, die auf einem Dichtelement 13 vorgesehen ist. Das Dichtelement 13 ist auf der inneren Leitung 8 der zweiten Kupplungshälfte 2 in Axialrichtung verschiebbar gelagert und wird durch eine Feder 15 in Richtung der ersten Kupplungshälfte 1 vorgespannt. Die Feder 15 wirkt auf einen Federteller 16, der über eine Isolierscheibe 17 mit einem elektrischen Kontakt 18 in Verbindung steht. Der erste Kontakt 18 ist im Wesentlichen zylindrisch ausgebildet und besitzt einen erweiterten Endabschnitt 19. An der ersten Kupplungshälfte 1 ist ein weiterer elektrischer Kontakt 20 über Isolierscheiben 21, 22 befestigt, um mit dem ersten Kontakt 18 der zweiten Kupplungshälfte 2 zusammenzuwirken. Der Rückstrom des kryogenen Mediums erfolgt durch Bohrungen 23 in einer Halterung 24, die in der äußeren Leitung 3 befestigt ist. Die elektrischen Kontakte 18, 20 sind über entsprechende Verbindungsleitungen 25, 26 mit nicht dargestellten Geräten verbunden.

Die äußeren Leitungen 3 beziehungsweise 7 sind durch außen angebrachte Vakuumisolierungen 27, 28 thermisch isoliert. Die Erzeugenden der konusförmigen Dichtflächen 9, 10; 11, 12 schließen mit der Achse der Leitungen 3, 4; 7, 8 einen Winkel α zwischen 6° und 12° ein, der hier etwa 10° beträgt. Die elektrischen Kontakte 18, 20 sind im Sinn einer Presspassung ausgelegt, bei der der Außendurchmesser eines Kontakts 18 um etwa 0,3 mm kleiner ist als der Innendurchmesser des anderen Kontakts 20. Weiters ist der Kontakt 20 auf seiner Innenseite durch vier nicht dargestellte Nuten strukturiert, die sich über zwei Drittel der Länge erstrecken.

Die Kupplungshälften 1, 2 sind über einen Bajonettverschluss 29 miteinander verbindbar, der aus einer Hülse 30 besteht, der die Vakuumisolierungen 27, 28 umgreift. Die Hülse 30 wird durch eine Feder 31 in einer Richtung, die von der zweiten Kupplungshälfte 2 weggerichtet ist, vorgespannt, wobei sich die Feder 31 auf einem Ring 32 abstützt, der mit der ersten Kupplungshälfte 1 fest verbunden ist. Zapfen 33, die mit der zweiten Kupplungshälfte 2 verbunden sind, greifen in Ausnehmungen 34 der Hülse 30 ein, um eine feste Verbindung zu gewährleisten. Die Zapfen 33 können auch als Feststellschrauben ausgebildet sein.

Die vorliegende Erfindung ermöglicht es, nicht nur eine hydraulisch dichte und sichere Verbindung für kryogenen Medien zu schaffen, sondern gleichzeitig auch eine sichere und haltbare elektrische Verbindung zu gewährleisten.

## Patentansprüche

1. Verbindungsanordnung für Leitungen (3, 4; 7, 8), die koaxial ineinander angeordnet sind, um den Hinstrom und Rückstrom eines kryogenen Mediums aufzunehmen, mit einer ersten und einer zweiten Kupplungshälfte (1, 2), die dichtend miteinander verbunden werden können, wobei eine innere Leitung (4; 8) in jeder Kupplungshälfte (1, 2) in einer ersten konusförmigen Dichtfläche (9; 10) endet, und wobei eine die innere Leitung (4; 8) umgebende äußere Leitung (3; 7) in jeder Kupplungshälfte (1, 2) in einer weiteren konusförmigen Dichtfläche (11; 12) endet, die radial außerhalb der ersten konusförmigen Dichtfläche (9; 10) angeordnet ist, **dadurch gekennzeichnet, dass** innerhalb der weiteren konusförmigen Dichtfläche (11; 12) in jeder Kupplungshälfte (1, 2) mindestens ein elektrischer Kontakt (18, 20) vorgesehen ist, welche Kontakte (18, 20) mit elektrischen Verbindungsleitungen (25, 26) zum Anschluss von Geräten verbunden sind.

2. Verbindungsanordnung nach Anspruch 1, **dadurch gekennzeichnet, dass** die elektrischen Kontakte (18, 20) koaxial zu den Leitungen (3, 4; 7, 8), angeordnet sind.

3. Verbindungsanordnung nach einem der Ansprüche 1 oder 2, **dadurch gekennzeichnet, dass** die elektrischen Kontakte (18, 20) im Wesentlichen zylindrisch ausgebildet sind.

4. Verbindungsanordnung nach einem der Ansprüche 1 bis 3, **dadurch gekennzeichnet, dass** eine erste Dichtfläche (10) auf einem Dichtelement (13) angeordnet ist, das in Axialrichtung verschiebbar und federnd vorgespannt mit der inneren Leitung (8) verbunden ist.

5. Verbindungsanordnung nach einem der Ansprüche 1 bis **4, dadurch gekennzeichnet, dass** die Kupplungshälften (1, 2) durch einen Bajonettverschluss (29) verbindbar sind.

6. Verbindungsanordnung nach Anspruch 5, **dadurch gekennzeichnet, dass** der Bajonettverschluss (29) eine Hülse (30) aufweist, die durch eine Feder (31) gegenüber einer Kupplungshälfte (1) vorgespannt ist.

7. Verbindungsanordnung nach einem der Ansprüche 1 bis 6, **dadurch gekennzeichnet, dass** die äußere Leitung (3, 7) durch eine Vakuumisolierung (27, 28) thermisch isoliert ist.

8. Verbindungsanordnung nach einem der Ansprüche 1 bis 7, **dadurch gekennzeichnet, dass** die konusförmigen Dichtflächen (9, 10; 11, 12) einen Winkel (α) zwischen 6° und 12° zur Achse der Leitungen (3, 4; 7, 8), aufweisen.

9. Verbindungsanordnung nach einem der Ansprüche 1 bis 8, **dadurch gekennzeichnet, dass** die elektrischen Kontakte (18, 20) eine Presspassung aufweisen und vorzugsweise mit einer strukturierten Oberfläche versehen sind.

## Claims

1. A connection assembly for conduits (3, 4; 7, 8) which are arranged coaxially inside one another to accommodate the supply flow and return flow of a cryogenic medium, with a first and a second coupling half (1,2) which can be connected to one another in a sealed manner, an inner conduit (4; 8) in each coupling half (1, 2) ending in a first conical sealing surface (9; 10) and an external conduit (3; 7) in each coupling half (1, 2), which encompasses the inner conduit (4; 8), ending in an additional conical sealing surface (11; 12) that is positioned outside the first conical sealing surface (9; 10) in the radial direction, **characterized in that** there is provided at least one electrical contact (18, 20) within the additional conical sealing surface (11; 12) in each coupling half (1, 2), said contacts (18, 20) being connected to electrical connecting lines (25, 26) that connect to instruments.

2. The connection assembly according to claim 1, **characterized in that** the electrical contacts (18, 20) are arranged coaxially relative to the conduits (3, 4; 7, 8).

3. The connection assembly according to one of the claims 1 or 2, **characterized in that** the electrical contacts (18, 20) are given a substantially cylindrical shape.

4. The connection assembly according to one of the claims 1 through 3, **characterized in that** a first sealing surface (10) is arranged on a sealing, element (13) that is axially slidable and resiliently biased and thus connected to the inner conduit (8).

5. The connection assembly according to one of the claims 1 through 4, **characterized in that** the coupling halves (1, 2) are connectable through a bayonet socket (29).

6. The connection assembly according to claim 5, **characterized in that** the bayonet socket (29) is provided with a case (30) that is biased by a spring (31 ) relative to a coupling half (1 ).

7. The connection assembly according to one of the claims 1 through 6, **characterized in that** the external conduit (3, 7) is thermally insulated by a vacuum insulation (27, 28).

8. The connection assembly according to one of the claims 1 through 7, **characterized in that** the conical sealing surfaces (9, 10; 11, 12) are positioned at an angle (α) from 6° to 12° to the axis of the conduits (3, 4; 7, 8).

9. The connection assembly according to one of the claims 1 through 8, **characterized in that** the electrical contacts (18, 20) are press fitted and are preferably provided with a structured surface.

## Revendications

1. Système de liaison de conduites (3, 4 ; 7, 8) disposées coaxialement les unes dans les autres pour recevoir le flux et le reflux d'un fluide cryogène, comprenant une première et une deuxième moitiés d'accouplement (1, 2) pouvant être assemblées de manière étanche l'une avec l'autre, une conduite intérieure (4 ; 8) dans chaque moitié d'accouplement (1, 2) débouchant dans une première surface d'étanchéité conique (9 ; 10), et une conduite extérieure (3 ; 7) entourant la conduite intérieure (4; 8) dans chaque moitié d'accouplement (1, 2) débouchant dans une autre surface d'étanchéité conique (11 ; 12) disposée radialement à l'extérieur de la première surface d'étanchéité conique (9 ; 10),
**caractérisé en ce qu'**
à l'intérieur de l'autre surface d'étanchéité conique (11 ; 12) dans chaque moitié d'accouplement (1, 2) au moins un contact électrique (18, 20) est prévu, lesquels contacts (18, 20) étant reliés à l'aide des lignes de liaison électriques (25, 26) pour raccorder des appareils.

2. Système de liaison selon la revendication 1,
**caractérisé en ce que**
les contacts électriques (18, 20) sont disposés coaxialement aux conduites (3, 4 ; 7, 8).

3. Système de liaison selon l'une des revendications 1 ou 2,
**caractérisé en ce que**
les contacts électriques (18, 20) sont formés essentiellement de manière cylindrique.

4. Système de liaison selon l'une des revendications 1 à 3,
**caractérisé en ce qu'**
une première surface d'étanchéité (10) est disposée sur un élément d'étanchéité (13), qui est relié en étant mobile dans le sens axial et élastiquement précontraint à la conduite intérieure (8).

5. Système de liaison selon l'une des revendications 1 à 4,
**caractérisé en ce que**
les moitiés d'accouplement (1, 2) peuvent être reliées à l'aide d'une fermeture à baïonnette (29).

6. Système de liaison selon la revendication 5,
**caractérisé en ce que**
la fermeture à baïonnette (29) présente une douille (30), qui est précontrainte par un ressort (31) par rapport à une moitié d'accouplement (1).

7. Système de liaison selon l'une des revendications 1 à 6,
**caractérisé en ce que**
la conduite extérieure (3, 7) est thermiquement isolée par une isolation à vide (27, 28).

8. Système de liaison selon l'une des revendications 1 à 7,
**caractérisé en ce que**
les surfaces d'étanchéité coniques (9, 10; 11, 12) présentent un angle (a) compris entre 6° et 12° par rapport à l'axe des conduites (3, 4 ; 7, 8).

9. Système de liaison selon l'une des revendications 1 à 8,
**caractérisé en ce que**
les contacts électriques (18, 20) présentent un ajustage serré et sont pourvus de préférence d'une surface structurée.
